Europäisches Patentamt

European Patent Office

Office européen des brevets

(11) Publication number: **0 552 380 A1**

## EUROPEAN PATENT APPLICATION
### published in accordance with Art. 158(3) EPC

(21) Application number: **92917677.4**

(22) Date of filing: **05.08.92**

(86) International application number:
**PCT/JP92/00998**

(87) International publication number:
**WO 93/03139 (18.02.93 93/05)**

(51) Int. Cl.5: **C12N 5/06, C12N 5/08,**
**C12N 11/02, C12M 3/00**

(30) Priority: **08.08.91 JP 199141/91**

(43) Date of publication of application:
**28.07.93 Bulletin 93/30**

(84) Designated Contracting States:
**DE FR GB**

(71) Applicant: **Kao Corporation**
**14-10, Nihonbashi Kayabacho 1-chome**
**Chuo-Ku Tokyo 103(JP)**
Applicant: **TOKYO WOMEN'S MEDICAL**
**COLLEGE**
**8-1, Kawata-cho, Shinjuku-ku**
**Tokyo 162(JP)**

(72) Inventor: **SAKAI, Hideaki**
**Room 401 Chisan Manshon Nakanoshima**
**553, Arimoto**
**Wakayama-shi Wakayama 640(JP)**
Inventor: **YOSHINO, Tomoko**

**5-2, Sekido 3-chome**
**Wakayama-shi Wakayama 641(JP)**
Inventor: **NAKAMURA, Koichi**
**2-9, Yamate**
**Funabashi-shi Chiba 273(JP)**
Inventor: **OKANO, Teruo**
**6-12-12, Konodai**
**Ichikawa-shi Chiba 272(JP)**
Inventor: **YAMADA, Noriko**
**Room 1-601 Maenocho Haitsu 6-10,**
**Maeno-cho**
**Itabashi-ku Tokyo 174(JP)**
Inventor: **SAKURAI, Yasuhisa**
**3-17-6, Eifuku**
**Suginami-ku Tokyo 168(JP)**

(74) Representative: **Vossius & Partner**
**Siebertstrasse 4 P.O. Box 86 07 67**
**W-8000 München 80 (DE)**

(54) **CELL CULTURE SUPPORT, PRODUCTION THEREOF, AND PRODUCTION OF CELL CLUSTER USING SAME.**

(57) A cell culture support comprising a base material to which cells adhere and a layer of a polymer having a critical temperature of dissolution in water of 0 to 80 °C applied to the surface of the base material in a coating weight of 2 to 80 mg/m$^2$; a process for the production of the support; and a process for the production of a cell cluster by culturing cells on the support and peeling off the culture product therefrom.

Field of the Invention

The present invention relates to a bed material for cell culture in the fields of biology, medical science, immunology and the like, a process for producing the same and a process for producing a cell aggregate using the same.

Prior Art

Hitherto, culturing cells have been conducted on the surface of glass or a surface-treated synthetic polymer. For example, a polystyrene which has been subjected to a surface treatment (e.g. γ ray irradiation, silicon coating, etc.) is used as a bed material for cell culture. Cultured cells on the bed are collected or detached from the surface of the bed by treating with a proteolysis enzyme (e.g. trypsin, etc.) or a chemical material.

In the treatment with the proteolysis enzyme or chemical material, however, following problems are associated: (1) Treating process is complicated and there are high possibility in introducing impurities. (2) The grown cells are denatured by the treatment and may hurt their inherent functions.

In order to overcome the above mentioned problems, the present inventors suggested a bed material used for cell culturing, from which cultured or grown cells can be collected or detached from the surface of the bed by simply changing an environmental temperature without treating with a proteolysis enzyme (e.g. trypsin, etc.) or a chemical material (e.g. EDTA, etc.) [Japanese Patent Kokai No. 2-211865 corresponding to U.S. Patent Application Ser. No. 07/817,954 to Teruo Okano et al.].

The cell aggregate obtained in this invention can be collected or detached by changing temperature, however, irregularity is sometimes observed on a polymer-coated surface on a support. For example, when cells during culturing are observed with a microscope, an irregular image of the surface and that of a cultured cells are doubled each other and, therefore, microscopic observation is not smoothly conducted, sometimes.

Object of the Invention

The object of the present invention is to provide a bed material for cell culture wherein the polymer-coated surface on the support has no irregularity (has smooth surface) and the state of cell culturing can be clearly observed by a microscope, and the collection or detachment of cells can be conducted easily and effectively, a process for producing the same and a process for producing a cell aggregate using the same, by developing the invention of the above cited publication.

It has been found that, by applying a specific amount of a homopolymer or copolymer on the bed material for cell culture suggested by the present inventors, the polymer-coated surface becomes smooth and irregularity is hardly observed by a microscope. Accordingly, the state of cell culturing can be clearly observed without doubling of the irregular image and the image of the cultured cells, as the case that the above suggested bed material for cell culture is used. On cell culturing, cells are uniformly adhered to the surface of the bed material and grown. Furthermore, the detached cells can be obtained in the aggregate state, as the case that the above bed material wherein the collection or detachment of the cultured cells can be efficiently conducted is used.

Summary of the invention

The present invention provides a bed material for cell culture comprising a cell adhesive support and a polymer layer coated on the surface of the support in a coating weight of 2 to 80 mg/m$^2$, said polymer layer being formed from a polymer having a critical solution temperature to water within the range of 0 to 80°C.

The present invention also provides a process for producing a bed material for cell culture which comprises applying a monomer solution comprising a monomer from which a polymer having a critical solution temperature to water within the range of 0 to 80°C is obtained and a solvent to the surface of a support such that a coating weight of a solvent component in the monomer solution becomes 3 to 100 ml/m$^2$ and then irradiating an electron beam.

The present invention further provides a process for producing a cell aggregate which comprises culturing cells using the bed material for cell culture and then detaching the cells.

Detailed Description of the Invention

The bed material for cell culture of the present invention is one that a surface of the support is coated with a specific polymer. The polymer has a critical solution temperature (hereinafter, sometimes, referred to as "T") of 0 to 80°C, preferably 10 to 50°C, more preferably 15 to 37°C. When the polymer having T of more than 80°C is used as a coating polymer, remarkable deterioration of activity of cells or death of cells are arisen at said temperature because a culture medium must be placed at a temperature higher than T of the polymer on the production of the cell aggregate of the present invention as described below. When the polymer having T of less than 0°C is used, remarkable deterioration of activity of cells is also arisen as described above because the culture medium must be placed at a temperature lower than 0°C. Further, the term "critical solution temperature" used herein means a temperature at which two kinds of substances which are divided into two layers are completely solved in each other to form an uniform layer. If the uniform layer is formed when heated, the critical solution temperature is called "upper critical solution temperature". If the uniform layer is formed when cooled, it is called "lower critical solution temperature".

The polymer constituting the polymer layer in the present invention is a homopolymer or copolymer obtained from a monomer which is selected from the group consisting of (meth)acrylamide, N-substituted- or N,N-disubstituted-(meth)acrylamide, alkyl vinyl ether and a mixture thereof. Examples of the substituent of nitrogen atom of (meth)acrylamide include an alkyl group having 1 to 8 carbon atoms, an alkoxyalkyl group having 2 to 8 carbon atoms, cycloalkyl group having 3 to 6 carbon atoms, a heterocyclic group containing nitrogen or oxygen atom (nitrogen atom of heterocycle may be that constituting an amide group of (meth)acrylamide) and the like. Further, other monomers may be further added to conduct copolymerization, in order to adjust a critical solution temperature depending upon a kind of grown cells, to enhance an interaction between the coating substance and bed material for cell culture, and to control the balance between hydrophilic nature and hydrophobic nature of the bed material. A graft or block copolymer of the above listed polymer used in the present invention and other polymers, or a mixture of the polymers used in the present invention and other polymers may be used. The polymer or copolymer used in the present invention may be crosslinked unless inherent properties of the polymer are adversely affected. As the suitable polymer used in the present invention, for example, there are poly(N-isopropyl acrylamide) (T = 32°C), poly(N-n-propyl acrylamide) (T = 21°C), poly(N-n-propyl methacrylamide) (T = 32°C), poly(N-ethoxyethyl acrylamide) (T = about 35°C), poly(N-tetrahydrofurfuryl acrylamide) (T = about 28°C), poly(N-tetrahydrofurfuryl methacrylamide) (T = about 35°C), poly(N,N-diethyl acrylamide) (T = about 35°C), as disclosed in Japanese Patent Kokai No. 2-211865. Examples of the other polymer include poly(N-ethyl acrylamide), poly(N-isopropyl methacrylamide), poly(N-cyclopropyl acrylamide), poly(N-cyclopropyl methacrylamide), poly(N-acryloylpyrrolidine), poly(N-acryloylpiperidine), polymethyl vinyl ether and the like.

The bed material for cell culture used in the present invention is obtained by coating the polymer on the support. The coating weight of the polymer is 2 to 80 mg/m$^2$, preferably 4 to 50 mg/m$^2$, more preferably 6 to 30 mg/m$^2$. When the coating weight of the polymer exceeds 80 mg/m$^2$, it becomes difficult for cells to adhere to the surface of the bed material for cell culture. On the contrary, when the coating weight is less than 2 mg/m$^2$, cells can adhere to the surface and conduct culturing, but it becomes impossible to collect or detach cells.

The coating weight can be determined, using Fourier transform infrared spectrophotometric analysis total reflection (FT-IR-ATR) method, analysis due to staining of the coated or non-coated part or labeling of fluorescent substance and surface analysis due to measurement of contact angle alone or in combination thereof. Among them, a method for determination of a coating weight according to FT-IR-ATR method wherein a polystyrene (Falcon 3002) Petri dish is used as the support and poly(N-isopropyl acrylamide) is used as the polymer to be coated is as follows.

Assuming that the thickness of the coating of the polymer is sufficiently than in comparison with a depth of penetration of IR ray and polystyrene as the support is an internal standard (the amount is always constant), a ratio of the amount of the coated polymer to that of polystyrene can be determined by the equation (1):

$$\text{Absorption intensity ratio} = \text{Abs}_{1640}/\text{Abs}_{1600}$$

wherein absorption intensity ratio is a ratio of the amount of the coated polymer to that of polystyrene, $\text{Abs}_{1640}$ is amide I (1640 cm$^{-1}$) absorption intensity derived from poly(N-isopropyl acrylamide) coated and $\text{Abs}_{1600}$ is benzene ring stretching (1600 cm$^{-1}$) absorption intensity derived from polystyrene.

If only a known amount of poly(N-isopropyl acrylamide) is applied on the surface of Falcon 3002 and a calibration curve relating to a coating weight and absorption intensity ratio is made by the above equation

3

(1), it becomes possible to determine the coating weight of the polymer of the bed material of the present invention from the calibration curve by measuring the absorption intensity ratio.

The support to be coated may be any material to which cells can adhere, and example thereof includes glass which is normally used for cell culture, modified glass, polymers (e.g. polystyrene, polymethyl methacrylate, etc.), ceramics, metal and the like. In that case, the support surface may be hydrophilized using a treating technique such as ozone treatment, plasma treatment, spattering and the like. The shape of the support is not limited, but typically Petri dish, plate, fiber, (porous) particle, any containers for cell culture (e.g. flask, etc.).

In the present invention, a homopolymer or copolymer having a critical solution temperature within the range of 0 to 80°C are coated on the surface of the cell adhesive support. An area of the part coated with the polymer layer is 40 to 100 % based on the total surface of the cell adhesive support, on observing the support surface from above. In view of detaching of cells, it is preferably 60 to 100 %, more preferably 70 to 100 %. The form thereof is not specifically limited, but typically pattern of line and space, polka-dot pattern, lattice pattern, pattern of special form or fine pattern wherein the above pattern are mixed. Further, the size of the coated part and the side shape thereof are not specifically limited. The arrangement of the coated and non-coated part may be regular or irregular, but preferably scattered.

The coating % can be determined, using elemental analysis due to X lay electron spectroscopy (XPS) method, analysis due to staining of the coated or non-coated part or labeling of fluorescent substance and surface analysis due to measurement of contact angle alone or in combination thereof. For example, when a polystyrene (Falcon 3002) Petri dish is used as the support and poly(N-isopropyl acrylamide) is used as the polymer, the coating % can be determined by measuring N atom (derived from amide bond) which is not present in polystyrene but in poly(N-isopropyl acrylamide) by XPS and calculating by the equation (2):

$$\text{Coating \%} = \frac{\text{N atom \% after coating}}{\text{N atom \% (12.5 \%) of P}} \times 100$$

wherein P is poly(N-isopropylamide).

According to the same manner as that described above, the coating % may be calculated based on the other atom (e.g. oxygen atoms, etc.) which is present in the polymer, instead of N atoms.

The polymer can be coated on the support using a chemical method and physical method described below alone or in combination. When the above monomer is used upon coating, the monomer may be in any state (e.g. gas, liquid, solid, etc.) at normal temperature. Further, when the polymer is used, the polymer may be in any state (e.g. gas, liquid, solid, etc.) at normal temperature. When these are bonded to the support by chemical reaction, electron beam (EB) irradiation, γray irradiation, ultraviolet irradiation, plasma treatment, corona treatment and the like can be used. When the support and coating material have a suitable reactive functional group, respectively, a normal organic reaction (e.g. radical reaction, ion reaction, etc.) can be used. In the method due to a physical interaction, the coating material per se or a combination of the coating material and a matrix compatible with the support is coated on the support, thus binding by physical absorption.

For example, the coating can also be conducted by applying a solution comprising a monomer and a suitable solvent on the support, followed by electron beam irradiation to polymerize the monomer. The solvent to be used is not specifically limited, and it may be any one which solves the monomer. It is preferred that the solvent has a boiling point of not more than 120°C, particularly 40 to 110°C under normal pressure. When the boiling point is too high, efficiency of polymerization by electron beam is deteriorated. Examples of the preferred solvent include methanol, ethanol, n- or i-propanol, 2- or n-butanol, water and the like, and one or more sorts of them may be used. Other solvents, for example, 1-pentanol, 2-ethyl-1-butanol, 2-butoxyethanol, ethylene (or diethylene) glycol or monoethyl ether thereof may be used in combination. To the solution, additives (e.g. sulphuric acid, sodium hydroxide, sodium chloride, etc.) may be formulated. The concentration of the monomer contained in the solution is not specifically limited, but it is preferably 10 to 70 % by weight, more preferably 20 to 60 % by weight, most preferably 30 to 55 % by weight in view of properties of the resulting bed material for cell culture and application properties of the above solution on the support.

The coating weight of the solution to the support is defined as the coating weight of a solvent component in the solution, for example, 3 to 100 ml/m$^2$, preferably 5 to 50 ml/m$^2$. When the coating weight of the solvent is less than 3 ml/m$^2$, application properties of the solution on the support is inferior. On the contrary, when it exceeds 100 ml/m$^2$, the surface of the resulting bed material does not become smooth, and it is not preferred. Further, the coating weight of the monomer is preferably 10 to 150 ml/m$^2$, particularly 15 to 70 ml/m$^2$.

The solution may be applied on the support by any known method, for example, coating method using spin coater or bar coater, spray coating method and the like.

After completion of the application, electron beam is irradiated on the support on which the solution has been applied to polymerize the monomer in the solution. The irradiation dose of electron beam is 5 to 40 Mrad, preferably 10 to 35 Mrad, more preferably 15 to 30 Mrad. When the dose is less than 5 Mrad, sufficient detaching property of cells is not obtained. On the contrary, it exceeds 40 Mrad, the support causes degradation (e.g. discoloration, cracking, etc.). Further, the irradiation dose of electron beam is determined by the equation:

Irradiation dose of electron beam = f x electron current (mA)/Line speed (m/min)

wherein f is an inherent scalar value to electron beam irradiation apparatus. Electron beam may be irradiated in one portion, or irradiated stepwise in plural portions. In the latter case, the total irradiation dose of electron beam is preferably 5 to 40 Mrad and the first irradiation dose of electron beam is preferably 1 to 10 Mrad, particularly 2 to 6 Mrad, in view of properties of the resulting bed material and smoothness of the surface of the bed material. Other conditions for electron beam irradiation is as follows. A degree of vacuum at the electron beam generating part is not more than 5 x 10$^{-4}$ Torr, an accelerating voltage of electron beam is 150 to 600 kV and an oxygen concentration at the irradiation part is preferably adjusted to not more than 2,000 ppm by introducing (dry) nitrogen gas or other inert gas.

The polymer is coated on the support by electron beam irradiation. After irradiation, a normal workup (e.g. rinsing, drying, sterilization, etc.) is conducted to obtain a bed material for cell culture of the present invention. The rinsing may be conducted using deionized water having a temperature enough to swell the polymer coated on the support, or conducted with applying ultrasonic.

A cell aggregate of the present invention is produced using the bed material for cell culture thus obtained. Cells which may be applied for the bed material for cell culture of the present invention is not specifically limited, and examples thereof include bovine aorta endothelial cell, bovine hepatocyte, rat hepatocyte, rat fibroblast, human vascular endothelial cell, human keratinocyte and the like. These cells can be obtained by a method known to the art. Cells are normally cultured at 0 to 50°C. In the case of using the bed material for cell culture of the present invention, culturing may be conducted within this temperature range. However, when the polymer has the upper critical solution temperature, it may be ccnducted at a temperature lower than the above upper critical solution temperature. On the contrary, when the polymer has the lower solution temperature, it must be conducted at a temperature higher than the above lower critical solution temperature. Other culturing conditions are not specifically limited and culturing may be conducted under the condition which is normally used in the art. For example, the culture medium containing a serum [e.g. fatal. calf serum (FCS), etc.] or that containing no serum may be used.

In order to collect or detach cultured cells from the bed material for cell culture after culturing, only an ambient temperature may be changed to a temperature higher than the upper critical solution temperature of the above polymer, preferably 10°C higher than the upper critical solution temperature, or a temperature lower than the lower critical solution temperature, preferably 10°C lower than the lower critical solution temperature. Cultured cells may be collected or detached in a culture medium solution in which cells have been cultured or other isotonic solution. In that case, in order to detach cultured cells effectively and easily, the bed material for cell culture may be slightly tapped or shaken or the culture medium may be agitated using a pipet.

In the bed material for cell culture of the present invention, the polymer is uniformly coated on the surface of the bed material in comparison with the above suggested bed material for cell culture. Therefore, cells in the culture medium can uniformly adhere to the surface of the bed material to grow. Accordingly, each cell on the bed material is cultured under more uniform conditions and each cell which is collected or detached from the bed material has a comparatively uniform function. In the case of collecting or detaching cells in the state of sheet or aggregate by the above means, the obtained sheet-like or bulk cell aggregate has no deficient part (the part at which no cells are present) in comparison with the above suggested bed material because cells can be uniformly cultured using the bed material of the present invention.

The function of the present invention will be illustrated by using an embodiment wherein N-isopropyl acrylamide is used as a monomer for preparing a coating polymer and polystyrene which is normally used as a Petri dish material for cell culture is used as a support.

As described above, the bed material for cell culture of the present invention is coated with a polymer of N-isopropyl acrylamide in a coating weight within a specific range and a coating % within a specific range, and irregularity is hardly arisen on the surface of the bed material (i.e. the surface of the coated polymer on the support). Further, a solvent solving N-isopropyl acrylamide is applied on the support in a coating weight within a specific range and, therefore, momentary evaporation or bumping of the solvent on electron beam irradiation can be avoided As a result, a thickness of a solution layer on the support becomes uniform and coating unevenness of the solution can be prevented. Accordingly, the bed material for cell culture of the present invention has little irregularity on the surface and, therefore, cells which are cultured on the bed material can uniformly adhere to the entire surface to grow. In that case, there is no disadvantage that cultured cells are hardly observed because of irregularity of the surface.

Poly(N-isopropyl acrylamide) thus coated on the support has a lower critical solution temperature of about 32°C in water. At a temperature higher than 32°C as the lower critical solution temperature, the poly-(N-isopropyl acrylamide) coating reduces its volume and excludes water molecules inside the coating and, therefore, the surface of the bed material shows hydrophobic. At a temperature lower than 32°C, the coating increases its volume and the volume fraction occupied by water molecules inside the coating and, therefore, the surface of the bed material becomes hydrophilic. According to the present invention, the surface of the bed material for cell culture reversibly changes hydrophilic to hydrophobic, vice versa, by simply controlling temperature, whereby, adherence of cells to the bed material is changed. Therefore, it becomes possible to detach the grown or cultured cells from the bed material by simply controlling temperature without destroying the cells.

The cells and aggregate thereof thus obtained in the present invention has the following advantages.
(1) The grown cells are not damaged by the chemical treatment and the inherent functions of the cells are not injured.
(2) The detached cells have no deficient part and they can maintain the aggregate state.
(3) Uniform cultured cells, each of which has an uniform function is obtained.
(4) The detached cells and aggregate thereof contain no impurities other than the culture medium.

Examples

The following Examples further illustrate the present invention in detail but are not to be construed to limit the scope thereof.

Examples 1 to 5

A Petri dish (available from Becton Dickinson Labware Co., as FALCON 3002) was employed as a support and the cells to be cultured are bovine aorta endothelial cells. In order to coat the surface of the Petri dish support, the surface of the Petri dish was firstly rinsed with isopropyl alcohol and dried under a nitrogen flow. A predetermined amount of a solution of N-isopropyl acrylamide in isopropyl alcohol having a predetermined concentration which was prepared by using a predetermined amount of a solvent shown in Table 1 was charged on the Petri dish, which was gently placed on a horizontal table and the solution was applied and spread thereon. It was then irradiated with electron beam of 25 Mrad one time. In that case, a degree of vacuum at the electron beam generating part was not more than $1 \times 10^{-6}$ Torr, an accelerating voltage of electron beam was 200 kV and an oxygen concentration at the irradiating part was not more than 1000 ppm (the same conditions are employed in the following Examples and Comparative Examples). After electron beam irradiation, the Petri dish was rinsed with ion-exchange water at 5°C to remove remaining monomers and homopolymers which were not bonded to the surface of the Petri dish, and then dried in a clean bench. It was further subjected to ethylene oxide (EO) gas sterilization and sufficiently deaerated to obtain a bed material for cell culture as a final product.

The coating weight on the surface of the resulting bed material for cell culture was determined by measuring an absorption intensity ratio from the integrated results (128 times) obtained using FT-IR-ATR method (used apparatus: JIR-RFX 3002 manufactured by Nihon Denshi Co.) according to the above equation (1) described in the present specification, followed by calculating by a calibration curve which has been made in advance. The calibration curve used is represented by the equation:

Coating weight (mg/m$^2$) = 37.6 x absorption intensity ratio

The results are shown in Table 2 below.

Further, a coating % of the polymer on the resulting bed material for cell culture was determined from an analysis value (angle of incidence: 90°) using XPS (used apparatus: ESCA 1000 manufactured by Schimazu Seisakusho Co.) according to the above equation (2) described in the present specification. The results are shown in Table 2.

Furthermore, smoothness of the coated surface was examined with a microscope whether irregularity is present on the surface or not. Results are shown in Table 2.

Bovine aorta endothelial cells were cultured at 37°C in 5 % carbon dioxide, using a Dulbecco's modified Eagle's medium (DMEM) containing 10 % fetal calf serum (FCS) on the obtained Petri dish. After growing cells, the Petri dish was cooled to 5°C and allowed to stand for 30 minutes to detach the cells. A collection % of the grown cells (number of cells collected by detaching / number of cells to be grown x 100) was calculated and the results are shown in Table 2.

In that case, in order to measure the number of cells collected by detaching and the number of cells to be grown, cells must be formed into pieces. Accordingly, the detached cell aggregate was cooled to 5°C and allowed to stand and then formed into pieces by trypsin-EDTA treatment to determined the number of cells collected by detaching. Further, to the obtained number of cells collected by detaching, the number of cells determined by forming the cells, which were not detached even by cooling at 5°C and standing, into pieces by trypsin-EDTA treatment was added to determine the number of cells to be grown.

Table 1

|  | Concentration of monomer solution (weight %) | Amount of monomer solution expanded (ml/m²) | Amount of solvent used (ml/m²) |
|---|---|---|---|
| Ex.1 | 60 | 47 | 19 |
| Ex.2 | 50 | 47 | 24 |
| Ex.3 | 40 | 47 | 29 |
| Ex.4 | 30 | 47 | 34 |
| Ex.5 | 20 | 47 | 39 |

Examples 6 and 7

According to the same manner as that described in Example 2 except that a Petri dish (available from Becton Dickinson Labware Co. as FALCON 3002) was used and an irradiation dose of electron beam was

7

20 Mrad (one time) (Example 6) and 35 Mrad (one time) (Example 7), respectively, a bed material for cell culture was obtained. A coating weight, a coating % and smoothness of the coated surface were examined according to the same manner as that described in Example 2. The results are shown in Table 2 below. On the bed material, bovine aorta endothelial cells were cultured according to the same manner as that described in Example 2, and the cultured cells were detached to determine a collection % of the grown cells. The results are shown in Table 2.

Examples 8 and 9

According to the same manner as that described in Example 3 except that a Petri dish (available from Becton Dickinson Labware Co. as FALCON 3002) was used and an irradiation dose of electron beam was 10 Mrad (one time) (Example 8) and 15 Mrad (one time) (Example 9), respectively, a bed meterial for cell culture was obtained. A coating weight, a coating % and smoothness of the coated surface were examined according to the same manner as that described in Example 3. The results are shown in Table 2 below. On the bed material, bovine aorta endothelial cells were cultured according to the same manner as that described in Example 3, and the cultured cells were detached to determine a collection % of the grown cells. The results are shown in Table 2.

Examples 10 and 11

According to the same manner as that described in Example 2 except that a Petri dish (available from Becton Dickinson Labware Co. as FALCON 3002) was used and a solvent solving N-isopropyl acrylamide was ethanol (Example 10) and water (Example 11), respectively, a bed material for cell culture was obtained. A coating weight, a coating % and smoothness of the coated surface were examined according to the same manner as that described in Example 2. The results are shown in Table 2 below. On the bed material, bovine aorta endothelial cells were cultured according to the same manner as that described in Example 2, and the cultured cells were detached to determine a collection % of the grown cells. The results are shown in Table 2.

Examples 12 and 13

According to the same manner as that described in Example 3 except that a Petri dish (available from Becton Dickinson Labware Co. as FALCON 3002) was used and N-ethoxyethyl acrylamide (Example 12) and N-n-propyl methacrylamide (Example 13) were used in place of N-isopropyl acrylamide, respectively, a bed material for cell culture was obtained. A coating weight, a coating % and smoothness of the coated surface were examined according to the same manner as that described in Example 3. The results are shown in Table 2 below. On the bed material, bovine aorta endothelial cells were cultured according to the same manner as that described in Example 3, and the cultured cells were detached to determine a collection % of the grown cells. The results are shown in Table 2.

Table 2

| | Coating weight (mg/m²) | Coating % | Smoothness of coated surface | Collection % of grown cells |
|---|---|---|---|---|
| Ex.1 | 28 | 100 | Smooth | >90 |
| Ex.2 | 12 | 99 | Smooth | >90 |
| Ex.3 | 9 | 75 | Smooth | >90 |
| Ex.4 | 7 | 55 | Smooth | >90 |
| Ex.5 | 6 | 44 | Smooth | >90 |
| Ex.6 | 10 | 85 | Smooth | >90 |
| Ex.7 | 18 | 100 | Smooth | >90 |
| Ex.8 | 4 | 61 | Smooth | >90 |
| Ex.9 | 5 | 66 | Smooth | >90 |
| Ex.10 | 15 | 100 | Smooth | >90 |
| Ex.11 | 9 | 79 | Smooth | >90 |
| Ex.12 | 10 | 81 | Smooth | >90 |
| Ex.13 | 9 | 74 | Smooth | >90 |

Comparative Example 1

According to the same manner as that described in Example 1 except that a Petri dish (available from Becton Dickinson Labware Co. as FALCON 3002) which was not subjected to a surface treatment was used as it is as a bed material for cell culture, an experiment was conducted. The results are shown in Table 3 below.

Comparative Example 2

According to the same manner as that described in Example 2 except that a Petri dish (available from Becton Dickinson Labware Co. as FALCON 3002) was used as a support, N-isopropyl acrylamide was not used, electron beam was irradiated under the same condition as that of Example 2 and the resulting bed material was used as it is, an experiment was conducted. The results are shown in Table 3 below.

Comparative Examples 3 and 4

According to the same manner as that described in Example 1 except that a Petri dish (available from Becton Dickinson Labware Co. as FALCON 3002) was used as a support and a monomer solution having a predetermined concentration prepared by using an amount of a solvent shown in Table 4 was used, an experiment was conducted. The results are shown in Table 3 below.

Table 3

| | Coating weight (mg/m²) | Coating % | Smoothness of coated surface | Collection % of grown cells |
|---|---|---|---|---|
| Comp. Ex.1 | 0 | 0 | Smooth | * |
| Comp. Ex.2 | 0 | 0 | Smooth | * |
| Comp. Ex.3 | 1 | 17 | Smooth | * |
| Comp. Ex.4 | 82 | 100 | Irregular | >90 |

*: Detachment and collection are impossible.

Table 4

| | Concentration of monomer solution (weight %) | Amount of monomer solution applied (ml/m²) | Amount of solvent applied (ml/m²) |
|---|---|---|---|
| Comp. Ex.3 | 0.5 | 8.0 | 8.3 |
| Comp. Ex.4 | 65 | $3.5 \times 10^2$ | $1.2 \times 10^2$ |

Examples 14 to 18

According to the same manner as that described in Example 3 except that a Petri dish (available from Becton Dickinson Labware Co. as FALCON 3002) was used and electron beam was irradiated in plural portions as shown in Table 5, a bed material for cell culture was obtained. A coating weight, a coating % and smoothness of the coated surface were examined according to the same manner as that described in Example 3. The results are shown in Table 7 below. On the bed material, bovine aorta endothelial cells were cultured according to the same manner as that described in Example 3, and the cultured cells were detached to determine. a collection % of the grown cells. The results are shown in Table 7.

Table 5

| | Irradiation dose of electron beam (Mrad) | | |
|---|---|---|---|
| | 1st irradiation | 2nd irradiation | 3rd irradiation |
| Ex.14 | 1 | 15 | 0 |
| Ex.15 | 3 | 20 | 0 |
| Ex.16 | 6 | 20 | 0 |
| Ex.17 | 10 | 25 | 0 |
| Ex.18 | 6 | 6 | 15 |

Examples 19 to 22

According to the same manner as that described in Example 15 except that a solution of N-isopropyl acrylamide in isopropyl alcohol having a predetermined concentration prepared by using a predetermined amount of a solvent shown in Table 6 was charged on a Petri dish (available from Becton Dickinson Labware Co. as FALCON 3002) in a predetermined amount shown in Table 6 and the Petri dish was gently placed on a horizontal table, a bed material for cell culture was obtained. A coating weight, a coating ratio and smoothness of the coated surface were examined according to the same manner as that described in Example 1. The results are shown in Table 7 below. On the bed material, bovine aorta endothelial cells here cultured according to the same manner as that described in Example 1, and the cultured cells were detached to determine a collection % of the grown cells. The results are shown in Table 7.

Table 6

| | Concentration of monomer solution (weight %) | Amount of monomer solution expanded (ml/m²) | Amount of solvent used (ml/m²) |
|---|---|---|---|
| Ex.19 | 60 | 47 | 19 |
| Ex.20 | 50 | 47 | 24 |
| Ex.21 | 45 | 47 | 27 |
| Ex.22 | 30 | 47 | 34 |

Examples 23 and 24

According to the same manner as that described in Example 16 except that a Petri dish (available from Becton Dickinson Labware Co. as FALCON 3002) was used and a solvent solving N-isopropyl acrylamide was ethanol (Example 23) and water (Example 24), respectively, a bed material for cell culture was obtained. A coating weight, a coating ratio and smoothness of the coated surface were examined according to the same manner as that described in Example 3. The results are shown in Table 7 below. On the bed material, bovine aorta endothelial cells were cultured according to the same manner as that described in Example 3, and the cultured cells were detached to determine a collection % of the grown cells. The results are shown in Table 7.

Examples 25 and 26

According to the same manner as that described in Example 17 except that a Petri dish (available from Becton Dickinson Labware Co. as FALCON 3002) was used and N-ethoxyethyl acrylamide (Example 25) and N-n-propyl methacrylamide (Example 26) was used in place of N-isopropyl acrylamide, respectively, a bed material for cell culture was obtained. A coating weight, a coating ratio and smoothness of the coated surface were examined according to the same manner as that described in Example 3. The results are shown in Table 7 below. On the bed material, bovine aorta endothelial cells were cultured according to the

12

same manner as that described in Example 3, and the cultured cells were detached to determine a collection % of the grown cells. The results are shown in Table 7.

Table 7

| | Coating weight (mg/m²) | Coating % | Smoothness of coated surface | Collection % of grown cells |
|---|---|---|---|---|
| Ex.14 | 5 | 65 | Smooth | >90 |
| Ex.15 | 7 | 73 | Smooth | >90 |
| Ex.16 | 10 | 86 | Smooth | >90 |
| Ex.17 | 12 | 98 | Smooth | >90 |
| Ex.18 | 15 | 100 | Smooth | >90 |
| Ex.19 | 29 | 100 | Smooth | >90 |
| Ex.20 | 11 | 95 | Smooth | >90 |
| Ex.21 | 9 | 74 | Smooth | >90 |
| Ex.22 | 8 | 80 | Smooth | >90 |
| Ex.23 | 11 | 85 | Smooth | >90 |
| Ex.24 | 6 | 69 | Smooth | >90 |
| Ex.25 | 12 | 99 | Smooth | >90 |
| Ex.26 | 11 | 98 | Smooth | >90 |

Comparative Example 5

According to the same manner as that described in Example 3 except that a Petri dish (available from Becton Dickinson Labware Co. as FALCON 3002) was used as a support, N-isopropyl acrylamide was not used, electron beam was irradiated under the same condition as that of Example 15 and the resulting bed material for cell culture was used as it is, an experiment was conducted. The results are shown in Table 8 below.

Comparative Examples 6 and 7

According to the same manner as that described in Example 15 except that a Petri dish (available from Becton Dickinson Labware Co. as FALCON 3002) was used as a support, a monomer solution having a predetermined concentration prepared by using a predetermined amount of a solvent shown in Table 9 was used, electron beam was irradiated under the same condition as that of Example 15 and the resulting bed material for cell culture was used as it is, an experiment was conducted. The results are shown in Table 8 below.

Table 8

| | Coating weight (mg/m²) | Coating % | Smoothness of coated surface | Collection % of grown cells |
|---|---|---|---|---|
| Comp. Ex.5 | 0 | 0 | Smooth | * |
| Comp. Ex.6 | 1 | 16 | Smooth | * |
| Comp. Ex.7 | 83 | 100 | Irregular | >90 |

*: Detachment and collection are impossible.

Table 9

| | Concentration of monomer solution (weight %) | Amount of monomer solution expanded (ml/m²) | Amount of solvent used (ml/m²) |
|---|---|---|---|
| Comp. Ex.6 | 0.5 | 8.0 | 8.3 |
| Comp. Ex.7 | 65 | $3.5 \times 10^2$ | $1.2 \times 10^2$ |

As is apparent from the results of Examples 1 to 5, when the coating weight is 2 to 80 mg/m², the coating % is 40 to 100 % and the amount of isopropyl alcohol which is a solvent in a N-isopropyl acrylamide solution expanded on the support surface is 3 to $1 \times 10^2$ ml/m², the surface of the resulting bed material for cell culture is smooth in a microscopic level as shown in Table 2 and, at the same time, the collection % of the grown cells of 90 % or more can be obtained. It is possible to realize the above phenomenon by any method of Examples 1 to 5. The resulting bed material is preferred in comparison with the above suggested bed material. It is also possible to realize the above phenomenon by changing an irradiation dose of electron beam (as shown in Examples 6 to 9), a kind of a solvent (as shown in Examples 10 and 11) or a kind of a monomer (as shown in Examples 12 and 13). It is also possible by irradiating electron beam in plural portions, as shown in Examples 14 to 26.

On the other hand, it was impossible for a non-treated FALCON 3002 to detach cells by simply decreasing a temperature, as shown in Comparative Example 1. As shown in Comparative Examples 2 and 5, it was impossible for a material wherein a monomer solution was not expanded and electron beam was irradiated. As shown in Comparative Examples 3 and 6, it was impossible for a material of which coating weight, coating % and amount of a solvent are not within the predetermined range to detach cells by simply decreasing a temperature, although cells were grown. As shown in Comparative Examples 4 and 7, in a material obtained by preparing using the solvent in an amount of not less than $1 \times 10^2$ ml/m², the collection of the grown cells was not less than 90 %. However, irregularity was observed on the coated surface by microscopic examination and the cell image during culturing and irregular image thereof were doubled each other and, therefore, it was a material having insufficient properties in view of easy cell observation.

14

Example 27

In order to confirm an injured degree of the detached cells obtained in Example 3, the cells were centrifuged at 600 G for 5 minutes and stirred sufficiently to form into pieces. And collected $2 \times 10^5$ cells were cultured again on a Petri dish (available from Becton Dickinson Labware Co. as FALCON 3002). Culturing process was the same as that of Example 3. The results are shown in Table 10 below.

Example 28

In order to confirm an injured degree of the detached cells obtained in Example 15, the cells were centrifuged at 600 G for 5 minutes and and stirred sufficiently to form into pieces. And collected $2 \times 10^5$ cells were cultured again on a Petri dish (available from Becton Dickinson Labware Co. as FALCON 3002). Culturing process was the same as that of Example 3. The results are shown in Table 10 below.

Comparative Example 8

The adhered cells cultured in Comparative Example 1 was treated with a 0.05 % trypsin-0.02 % EDTA solution to detach them. Then, in order to confirm an injured degree of the detached cells, the cells were centrifuged at 600 G for 5 minutes to collect $2 \times 10^5$ cells, which were then cultured again on a Petri dish (available from Becton Dickinson Labware Co. as FALCON 3002). Culturing process was the same as that of Example 1. The results are shown in Table 10.

Table 10

|  | Number of cells when culturing started | Number of cells after 4 days |
|---|---|---|
| Ex.27 | $2 \times 10^5$ | $2 \times 10^6$ |
| Ex.28 | $2 \times 10^5$ | $2 \times 10^6$ |
| Comp. Ex.8 | $2 \times 10^5$ | $1 \times 10^6$ |

As to the injured degree of the detached cells, Examples 27 and 28 could grow 10 times more than the starting point as shown in Table 10, but Comparative Example 8 could grow only 5 times more. This shows that an injured degree of the detached cells of the present invention is smaller than that of a conventional one.

Example 29

In order to confirm uniformity of a function of the detached cells obtained in Example 2, the resulting sheet-like cell aggregate was divided into 4 pieces, which were sufficiently stirred to collect $2 \times 10^5$ cells. Then, they were cultured again on a Petri dish (available from Becton Dickinson Labware Co. as FALCON 3002). Culturing process was the same as Example 2. The function of cells was evaluated by determining an amount of $\alpha$-keto • FI $\alpha$ as metabolite of prostacylin using a radioimmunoassay method after culturing for one day. Each activity of the divided cell aggregate is shown in Table 11, respectively.

Example 30

In order to confirm uniformity of a function of the detached cells obtained in Example 16. the resulting sheet-like cell aggregate was divided into 4 pieces. Following operations were the same as those of Example 29. Each activity of the divided cell aggregate is shown in Table 11, respectively.

Comparative Example 9

The adhered cells cultured in Comparative Example 1 [a sheet-like cell aggregate can not be obtained at a low temperature and, therefore, an activity can be measured at only one point (average value) in an entire Petri dish] were treated with a 0.05 % trypsin-0.02 % EDTA solution to detach them. In order to confirm uniformity of an injured degree of the detached cells, the cells were centrifuged at 600 G for 5 minutes to collect $2 \times 10^6$ cells, which were then cultured again on a Petri dish (available from Becton Dickinson Labware Co. as FALCON 3002). Culturing process and activity measurement were the same as those of Example 29. The results are shown in Table 11.

Table 11

| PGFIα amount (ng/10⁵ cells) | | | | |
|---|---|---|---|---|
| | Split 1 | Split 2 | Split 3 | Split 4 |
| Ex. 29 | 1.2 | 1.2 | 1.2 | 1.2 |
| Ex.30 | 1.1 | 1.1 | 1.1 | 1.1 |
| Comp. Ex.9 | 0.17 | - | - | - |

As is apparent from Table 11, an activity of the detached cells of Examples 29 and 30 showed 6 to 7 times as that of Comparative Example 9. In Examples 29 and 30, each activity of splits 1 to 4 was also the same as the above. This shows that an injured degree of the detached cells of the present invention is smaller than that of a conventional one and culturing can be conducted uniformly on the surface of a bed material coated with a polymer.

According to the bed material for cell culture of the present invention, cultured cells can be collected or detached from a bed material by simply changing an ambient temperature without treating with a proteolysis enzyme (e.g. trypsin, etc.) or a chemical material (e.g. EDTA, etc.) and, therefore, there are following advantages.

(1) The process is simplified.

(2) Possibility of impurities is completely disappeared.

(3) The grown cells are not damaged by the chemical treatment.

(4) The inherent functions of the cells are not injured.

(5) The detached cells can maintain the aggregate state.

(6) The cells can be repeatedly collected or detached.

Further, the bed material for cell culture of the present invention has a smooth surface and have no irregularity and, therefore, the cell image and irregular image are not doubled each other by microscopic examination and cultured cells can be observed with a microscope clearly and easily. Furthermore, in the conventional bed material for cell culture, a function of collection or detachment extremely depend upon it's surface state. However, a function is uniformly developed without being influenced by the surface state according to the present invention and, therefore, a quality of the product becomes stable and it's yield becomes good and, at the same time, it becomes possible to select suitable monomer species/amount according to a kind of cells.

**Claims**

1. A bed material for cell culture comprising a cell adhesive support and a polymer layer coated on the surface of the support in a coating weight of 2 to 80 mg/m$^2$, said polymer layer being formed from a polymer having a critical solution temperature to water within the range of 0 to 80°C.

2. The bed material according to claim 1, wherein said critical solution temperature is within the range of 10 to 50°C.

3. The bed material according to claim 1, wherein said critical solution temperature is within the range of 15 to 37°C.

4. The bed material according to claim 1, wherein said polymer layer mainly comprises a homopolymer or copolymer of a monomer which is selected from the group consisting of (meth)acrylamide, N-substituted- or N,N-disubstituted-(meth)acrylamide, alkyl vinyl ether and a mixture thereof.

5. The bed material according to claim 1, wherein said coating weight is 4 to 50 mg/m$^2$.

6. The bed material according to claim 1, wherein said cell adhesive support is prepared from glass, polystyrene, polymethyl methacrylate, ceramics or metal.

7. The bed material according to claim 1, wherein the surface of said cell adhesive support is subjected to a hydrophilization treatment.

8. The bed material according to claim 1, wherein an area of the part coated with the polymer layer is 40 to 100 % based on that of the support, on observing the support surface from above.

9. The bed material according to claim 4, wherein said polymer layer is prepared from a polymer selected from the group consisting of poly(N-isopropyl acrylamide), poly(N-n-propyl acrylamide), poly(N-n-propyl methacrylamide), poly(N-ethoxyethyl acrylamide), poly(N-tetrahydrofurfuryl acrylamide), poly(N-tetrahydrofurfuryl methacrylamide), poly(N,N-diethyl acrylamide), poly(N-ethyl acrylamide), poly(N-isopropyl methacrylamide), poly(N-cyclopropyl acrylamide), poly(N-cyclopropyl methacrylamide), poly-(N-acryloylpyrrolidine), poly(N-acryloylpiperidine) and polymethyl vinyl ether.

10. A process for producing a bed material for cell culture which comprises applying a monomer solution comprising a monomer from which a polymer having a critical solution temperature to water within the range of 0 to 80°C is obtained and a solvent to the surface of a support such that a coating weight of a solvent component in the monomer solution becomes 3 to 100 ml/m$^2$ and then irradiating an electron beam.

11. The process according to claim 10, wherein said solvent has a boiling point of 120°C or less under normal pressure.

12. The process according to claim 10, wherein a total irradiation dose of the electron beam is 5 to 40 Mrad.

13. The process according to claim 10, wherein the electron beam is irradiated stepwise in plural portions.

14. The process according to claim 10, wherein said monomer is selected from the group consisting of (meth)acrylamide, N-substituted- or N,N-disubstituted (meth)acrylamide, alkyl vinyl ether and a mixture thereof.

15. The process according to claim 10, wherein said polymer layer is prepared from a polymer selected from the group consisting of poly(N-isopropyl acrylamide), poly(N-n-propyl acrylamide), poly(N-n-propyl methacrylamide), poly(N-ethoxyethyl acrylamide), poly(N-tetrahydrofurfuryl acrylamide), poly(N-tetrahydrofurfuryl methacrylamide), poly(N,N-diethyl acrylamide), poly(N-ethyl acrylamide), poly(N-isopropyl methacrylamide), poly(N-cyclopropyl acrylamide), poly(N-cyclopropyl methacrylamide), poly-(N-acryloylpyrrolidine), poly(N-acryloylpiperidine) and polymethyl vinyl ether.

16. The process according to claim 10, wherein said coating weight of the polymer layer is 2 to 80 mg/m$^2$.

17. A process for producing a cell aggregate which comprises culturing cells using the bed material for cell culture according to claim 1 and then detaching the cells.

# INTERNATIONAL SEARCH REPORT

International Application No PCT/JP92/00998

## I. CLASSIFICATION OF SUBJECT MATTER (if several classification symbols apply, indicate all) ⁶

According to International Patent Classification (IPC) or to both National Classification and IPC

Int. Cl⁵  C12N5/06, 5/08, 11/02, C12M3/00

## II. FIELDS SEARCHED

### Minimum Documentation Searched ⁷

| Classification System | Classification Symbols |
|---|---|
| IPC | C12N5/06-5/28, C12N11/02-11/12, C12M3/00, B01J20/22-20/26 |

### Documentation Searched other than Minimum Documentation to the Extent that such Documents are Included in the Fields Searched ⁸

BIOSIS

## III. DOCUMENTS CONSIDERED TO BE RELEVANT ⁹

| Category* | Citation of Document, ¹¹ with indication, where appropriate, of the relevant passages ¹² | Relevant to Claim No. ¹³ |
|---|---|---|
| Y | JP, A, 2-211865 (Kao Corp. and another), August 23, 1990 (23. 08. 90), & EP, A, 382214 | 1-17 |
| Y | JP, A, 2-504221 (Bey Mikael), December 6, 1990 (06. 12. 90), Particularly, lower left column, page 5 & WO, A, 88/08448 & EP, A, 355112 | 1-17 |
| A | JP, B2, 61-59177 (Kogyo Gijutsuin-cho), December 15, 1986 (15. 12. 86), (Family: none) | 1-17 |
| A | JP, A, 62-6682 (Canon Inc.), January 13, 1987 (13. 01. 87), & US, A, 4975375 | 1-17 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| November 3, 1992 (03. 11. 92) | November 24, 1992 (24. 11. 92) |

| International Searching Authority | Signature of Authorized Officer |
|---|---|
| Japanese Patent Office | |

Form PCT/ISA/210 (second sheet) (January 1985)